Europäisches Patentamt

(19) European Patent Office

Office européen des brevets

(11) Numéro de publication : **0 266 383 B1**

(12) # FASCICULE DE BREVET EUROPEEN

(45) Date de publication du fascicule du brevet :
**06.11.91 Bulletin 91/45**

(51) Int. Cl.⁵ : **A61F 11/00, A61M 3/00**

(21) Numéro de dépôt : **87902551.8**

(22) Date de dépôt : **21.04.87**

(86) Numéro de dépôt international :
**PCT/FR87/00131**

(87) Numéro de publication internationale :
**WO 87/06456 05.11.87 Gazette 87/24**

(54) **DISPOSITIF DE NETTOYAGE D'UNE CAVITE, NOTAMMENT LA CAVITE AURICULAIRE.**

(30) Priorité : **21.04.86 FR 8605840**

(43) Date de publication de la demande :
**11.05.88 Bulletin 88/19**

(45) Mention de la délivrance du brevet :
**06.11.91 Bulletin 91/45**

(84) Etats contractants désignés :
**AT BE CH DE FR GB IT LI LU NL SE**

(56) Documents cités :
**US-A- 3 398 743**
**US-A- 4 044 757**
**US-A- 4 244 377**

(73) Titulaire : **DENIS, Alain**
**Jardins de Vallauris Avenue G. Clémenceau**
**F-06220 Vallauris (FR)**

(72) Inventeur : **DENIS, Alain**
**704, chemin du Cannet**
**F-06220 Vallauris (FR)**
Inventeur : **DUMOUCHEL, Lionel**
**14, allée des Marronniers Lotissement La**
**Guieterie**
**F-78470 Saint Rémy les Chevreuse (FR)**

(74) Mandataire : **Hasenrader, Hubert et al**
**Cabinet BEAU DE LOMENIE 55, rue**
**d'Amsterdam**
**F-75008 Paris (FR)**

EP 0 266 383 B1

## Description

L'invention concerne un dispositif hygiénique pour le nettoyage d'une cavité, notamment une cavité organique (conduit auditif, narines, etc.). Elle concerne plus spécialement, mais non exclusivement, le lavage des oreilles par injection sous pression de produits liquides (eau chaude, eau savonneuse, sérum physiologique) en vue d'extraire les bouchons de cérumen.

Actuellement, l'utilisation fréquente des produits tels que des cotons-tiges a des conséquences sérieuses sur les conduits auditifs de leurs utilisateurs. Ces cotons-tiges bourrent souvent les débris de cérumen contre le tympan jusqu'à former un bouchon de cérumen et à diminuer l'acuité auditive ; ils peuvent perforer de manière traumatique le tympan et le coton peut se bloquer dans le conduit auditif externe.

D'un autre côté, ces cotons-tiges utilisés trop fréquemment éliminent trop rapidement le cérumen et peuvent dessécher le conduit auditif avec les conséquences que l'on connaît.

Lorsque le bouchon de cérumen est, par sa taille, trop gênant, le patient doit demander l'intervention d'un médecin spécialiste. Actuellement, celui-ci a à sa disposition une pompe à injection volumineuse dont l'extrémité d'un embout est placé dans le récipient du liquide à instiller, l'autre extrémité est placée dans le conduit auditif. D'une main, le médecin doit donner quelques coups de poire ou de pompe en surveillant la pression sur la pompe, de l'autre diriger l'extrémité du tuyau qui est dans le conduit auditif pour que le jet soit le plus efficace possible pour décoller et détruire le bouchon de cérumen. Ces différentes actions sont malaisées et ne peuvent être effectuées sans danger que par des professionnels. Enfin souvent les tuyaux sont désemmanchés et la pompe envoie le liquide à l'extérieur.

On connaît par le document FR-A-2 526 656 un laveur de conduits auditifs, à eau collectée et recyclée. Le laveur est composé d'un tube d'injection débouchant dans la cavité auriculaire ; un bocal est appliqué autour de l'oreille de manière à collecter l'eau qui s'écoule de l'oreille ; une tubulure prise sur le bocal ramène l'eau en amont du tube d'injection, une poire étant utilisée pour injecter l'eau recyclée, dont le sens de circulation est défini par des clapets anti-retour placés sur le tube d'injection et dans la tubulure de recyclage.

Ce système n'est pas hygiénique dans la mesure où l'eau usée est recyclée. Il est de plus d'un usage mal commode et est encombrant. L'oreille sort du traitement mouillée extérieurement, ce qui est aussi un inconvénient.

Le brevet US-A 3,398,743 décrit un appareil d'irrigation d'un organe visqueux comprenant notamment un injecteur (poire manuelle) présentant une pompe et un conduit d'injection ayant un orifice de sortie et destiné à amener le liquide propre à l'organe visqueux, une première chambre destinée à contenir le liquide propre et reliée au conduit d'injection, un conduit de récupération du liquide usé ayant un orifice d'entrée et, une seconde chambre destinée à recevoir le liquide usé récupéré par le conduit de récupération. Mais dans cet appareil, lorsque la poire est comprimée, une pression positive est produite dans le conduit d'injection. Puisque le liquide usé passe par le même tronçon extrême de conduit que le liquide refoulé en étant arrêté par un clapet de retenue, le liquide usé ne peut s'évacuer tant que la compression de la poire est maintenue. La surpression dans l'organe irrigué qui peut en résulter rend cet appareil trop dangereux pour le nettoyage d'une cavité auriculaire. L'invention a pour but de proposer un dispositif de nettoyage commode, hygiènique, ne présentant pas les inconvénients précités.

Le dispositif pour le nettoyage d'une cavité auriculaire que propose la présente invention est du type comportant un injecteur présentant une pompe et un conduit d'injection ayant un orifice de sortie et destiné à amener le liquide propre à la cavité, une première chambre destinée à contenir le liquide propre et reliée au conduit d'injection, un conduit de récupération du liquide usé ayant un orifice d'entrée et, une seconde chambre destinée à recevoir le liquide usé récupéré par le conduit de récupération.

Selon l'invention, ce dispositif est caractérisé en ce que le conduit de récupération est agencé de sorte que le liquide usé s'écoule par gravité de la cavité auriculaire à travers ce dernier jusqu'à la seconde chambre et présente une section supérieure à celle du conduit d'injection, et en ce qu'il comprend, en outre, un moyen d'étanchéité de ladite cavité, moyen traversé par le conduit d'injection et par le conduit de récupération, l'orifice de sortie du conduit d'injection et l'orifice d'entrée du conduit de récupération étant sensiblement adjacents et débouchant à l'intérieur de la cavité, lorsque le dispositif est en position de nettoyage.

Grâce à cette disposition, tout en évitant une surpression dans la cavité auriculaire, on assure un nettoyage efficace de celle-ci et une récupération correcte et hygiénique du liquide usé et des débris du bouchon de cerumen.

Pour que les orifices soient ainsi adjacents, le conduit de récupération et le conduit d'injection sont placés intérieurement l'un à l'autre, ou de manière contiguë, au moins dans la région de leur extrémité au niveau desdits orifices.

Il est avantageux que le conduit de récupération et le conduit d'injection soient adjacents sur au moins une partie terminale en amont du moyen d'étanchéité, de manière à les regrouper facilement dans un entourage commun unique, bec, sonde ou analogue, rigide ou souple, dont l'accès à la cavité est ainsi facilité.

Le moyen d'étanchéité peut être constitué par une jupe en paroi souple susceptible de se plaquer contre la cavité sous l'effet de la pression du liquide injecté.

D'une manière préférée, le moyen d'étanchéïté est constitué par un bouchon en matériau légèrement élastique, plus large que le col de la cavité à fermer, et qu'on applique contre ledit col. Les orifices du tube d'injection et du tuyau de récupération peuvent être formés directement.dans ce bouchon.

Avantageusement, la chambre contenant le liquide à injecter coopère avec la pompe (par exemple électrique, ou manuelle), ou même constitue partiellement celle-ci : elle est formée d'une enceinte à volume déformable, dans laquelle le tube d'injection prend naissance.

La déformation du volume peut être due à la déformation des parois de l'enceinte elle-même : la chambre peut être une poire, une chambre à soufflets ou analogue.

La déformation peut être due au déplacement relatif d'un piston dans l'enceinte : toutes pompes de ce type conviennent à l'invention.

La chambre contenant le liquide à instiller peut être pourvue d'un bouchon de remplissage, comportant une soupape anti-retour empêchant le liquide de sortir et permettant à l'air d'entrer.

Selon d'autres formes de réalisation, il peut être préféré que la chambre ne soit remplie qu'à la fabrication et ne puisse l'être ensuite, de manière à n'être utilisée qu'une fois et garantir des conditions d'hygiène strictes.

La chambre de récupération du liquide usé peut aussi comporter un bouchon permettant à l'air seul de s'échapper.

Il est à noter que le tuyau de récupération présente une section supérieure à celle du tube d'injection, notamment au niveau de leurs orifices respectifs, de manière à permettre un accès facile aux débris de bouchon de cérumen.

D'autres caractéristiques et avantages de l'invention ressortiront de la description suivante de plusieurs modes de réalisation. Il sera fait référence aux dessins annexés sur lesquels :

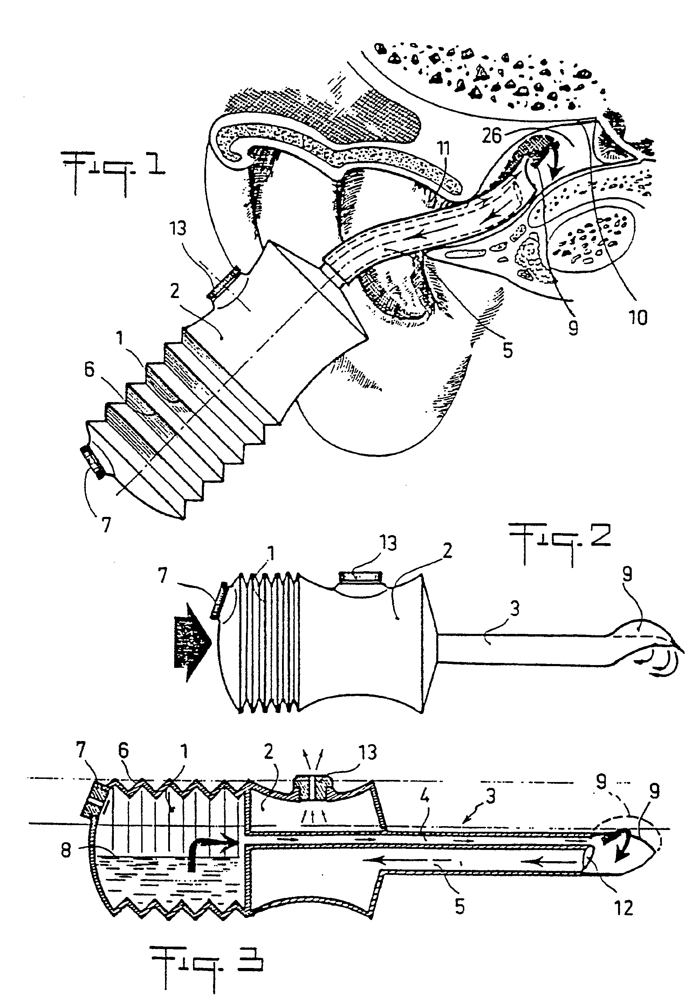

. la figure 1 est une vue en coupe partielle de l'oreille externe et de l'oreille interne, mettant en évidence la mise en place d'un premier mode de réalisation du dispositif selon l'invention.

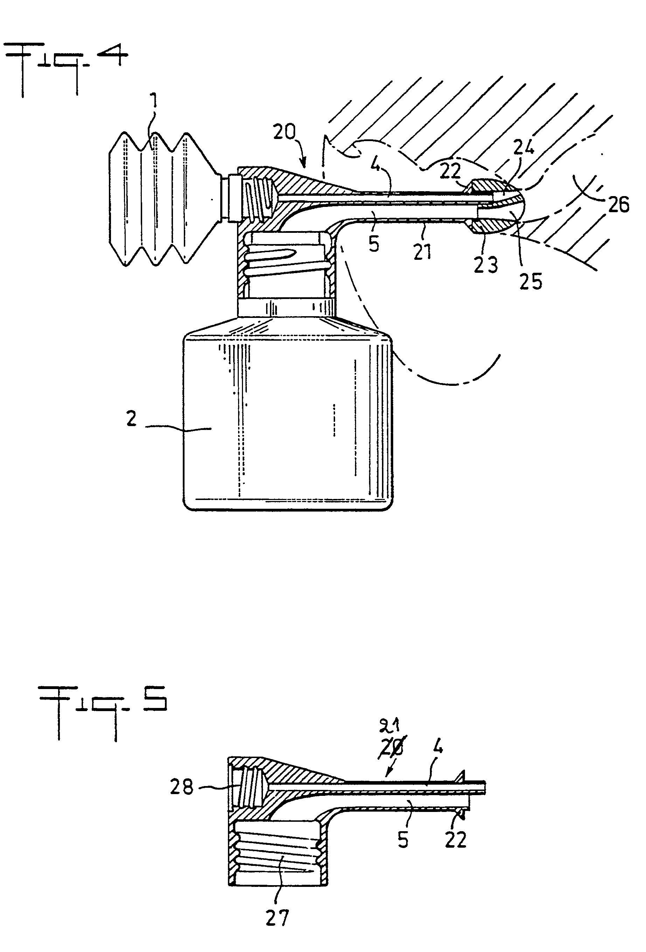

. la figure 2 est une vue de côté du dispositif de la figure 1.

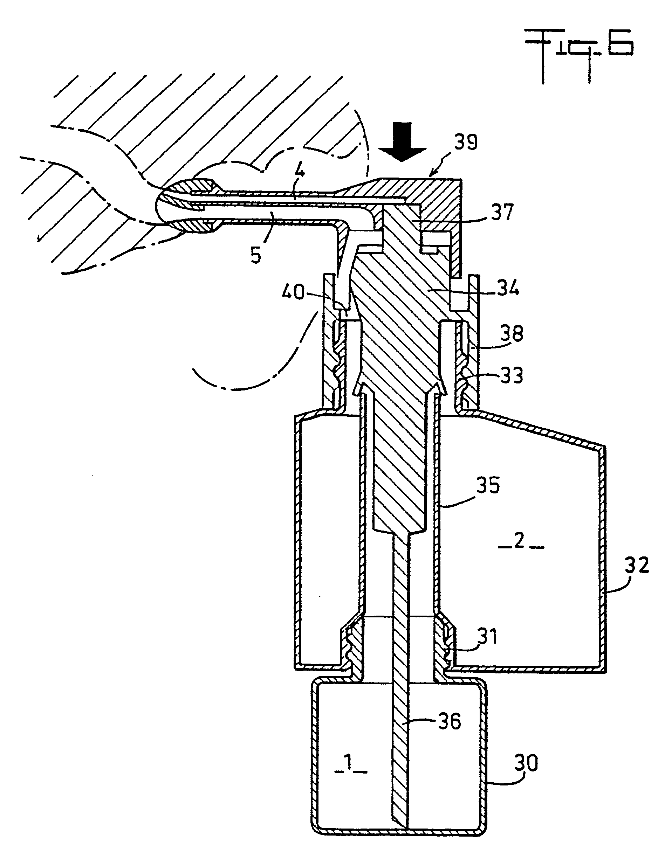

. la figure 3 est une vue en coupe du dispositif de la figure 2.

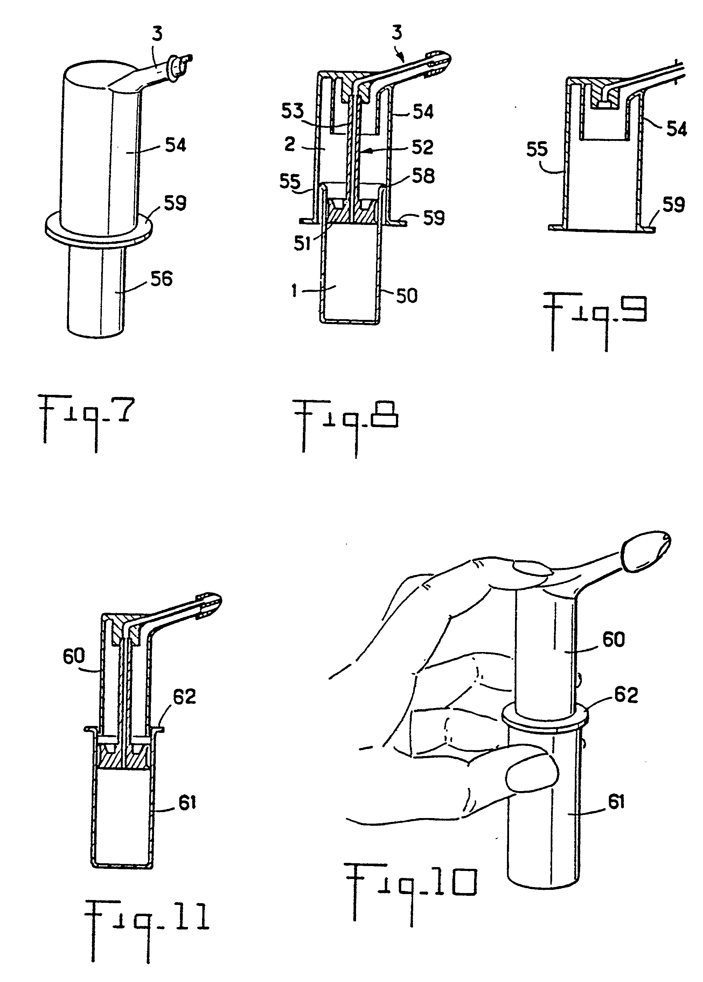

. la figure 4 est une vue avec coupe partielle d'un deuxième mode de réalisation.

. la figure 5 montre en coupe un détail (le bec) du dispositif de la figure 4.

. la figure 6 est une vue en coupe partielle d'un troisième mode de réalisation.

. la figure 7 est une vue en perspective d'un quatrième mode de réalisation (l'embout n'est pas montré).

. la figure 8 est une vue en coupe du dispositif de la figure 7.

. la figure 9 est une vue en coupe du diffuseur du dispositif précédent.

. la figure 10 est une vue en perspective d'un cinquième mode de réalisation.

. la figure 11 est une vue en coupe du dispositif de la figure 10.

Le dispositif des figures 1 à 3 est composé de deux chambres grossièrement cylindriques 1 et 2 accolées par une base commune de manière à former un bloc unitaire, et d'une sonde-souple 3, partant du bloc, renfermant deux tubes emboîtés définissant un conduit d'injection 4 et un conduit de récupération 5 et décrits par la suite respectivement en tant que tube 4 et tuyau 5.

La chambre 1 contient le liquide à instiller 8, dont le remplissage s'effectue grâce à une ouverture fermée par un bouchon à soupape 7. Les parois latérales 6 de la chambre 1 forme un soufflet dont l'écrasement manuel permet au liquide 8 de s'échapper à travers le tube 4 dont l'extrémité amont est prise sur la base commune entre les deux chambres 1 et 2. Le tube 4, en matériau très souple, traverse la chambre 2 avant de rejoindre la sonde 3.

La chambre 2, destinée au liquide de récupération, est rigide ou semi-rigide et est conformée pour servir commodément de prise à des doigts de la main afin d'écraser le soufflet de la chambre 1. Un bouchon 13 permet la sortie de l'air, lorsque la chambre 2 se remplit de liquide usagé.

A l'extrémité de la sonde 3, le moyen d'étanchéïté représenté est une jupe 9 en matière très souple entourant au moins partiellement l'extrémité de sonde. Lorsque le liquide à instiller s'échappe sous pression de l'orifice de sortie du tube 4, il repousse la jupe 9 qui obstrue partiellement son passage et vient la plaquer contre la paroi interne (la voûte supérieure 10 en l'occurrence) du conduit auditif. Le liquide usé retourne par écoulement gravitaire dans le tuyau de récupération 5 jusqu'à la chambre 2 ; pour faciliter ce retour, l'orifice d'entrée 12 du tube de récupération est largement ouvert et situé à la partie inférieure de la sonde lorsque celle-ci est en position correcte.

La jupe souple 9 permet d'adapter l'étanchéïté de la sonde aux différents diamètres de conduits auditifs et de la rapprocher au plus près du bouchon éventuel de cérumen.

Au lieu d'être emboîtés l'un dans l'autre, le tube 4 et le tuyau 5 pourraient être simplement parallèles et contigus tout le long de la sonde 3.

Le mode de réalisation des figures 1 à 3 peut être modifié en remplaçant le bloc des deux chambres par un autre agencement équivalent, en prévoyant notamment d'autres systèmes de pompes manuelles à pistons actionnés par leviers ou poussoirs, ou des pompes électriques du type, par exemple, de celles qu'utilisent les appareils de distribution d'eau sous pression pour le lavage des dents.

A la place de l'extrémité spécifique en jupe souple de la sonde 3, il est possible de terminer la sonde 3 avec des embouts intracanalaires épousant parfaitement cette partie du conduit auditif, comme par exemple les prothèses internes pour malentendants, et comportant deux orifices : un orifice supérieur par où arrive le liquide sous pression et un orifice inférieur, plus large, par où le liquide instillé usé revient.

Il est aussi possible de prévoir un embout ne pénétrant pas dans le conduit interne de l'oreille, mais fermant celui-ci au niveau du col de sortie.

Ainsi, selon le mode de réalisation de la figure 4, le dispositif comporte une chambre de récupération 2 réalisée sous forme d'un flacon récepteur dont le col comporte des filets de vissage permettant la fixation d'un bec 20. Celui-ci comporte essentiellement une partie de sonde rectiligne rigide 21 renfermant dans sa section inférieure le tuyau de récupération 5 et dans sa section supérieure le tube d'injection 4. La sonde possède près de son extrémité avale un épaulement périphérique 22 servant de butée à l'embout 23. Le tube d'injection 4 se prolonge en aval légèrement au delà du tuyau 5, de manière à faciliter la mise en place de l'embout 23.

Ce dernier comporte deux alésages intérieurs 24,25 correspondant à et prolongeant respectivement le tube 4 et le tuyau 5 de sorte qu'on peut considérer que les orifices d'entrée du tuyau et de sortie du tube sont formés directement par l'embout. L'embout 23 est de forme sensiblement semi-ovoïde, et est en matière légèrement élastique pour venir s'adapter sur le col de sortie du conduit auditif 26 et former par simple pression une étanchéïté. Une matière caoutchouteuse, éventuellement traitée pour résister aux liquides utilisés, convient.

A l'intérieur du bec 21, le tuyau 5 débouche dans une chambre inférieure filetée 27 s'adaptant sur le col du flacon 2, tandis que le tube 4 est pris en amont sur une chambre latérale filetée 28 dans laquelle se visse le col fileté de la chambre à soufflets 1, contenant le principe actif.

Le mode de réalisation de la figure 6 diffère de celui des figures 4 et 5 essentiellement par la disposition relative des chambres et la constitution de la pompe.

Ici la chambre 1 contenant le principe actif est constituée par un flacon rigide 30 à col fileté 31, sur lequel se visse un flacon récepteur 32 formant la chambre rigide de récupération 2, comportant lui-même un col fileté 33 servant à visser une jupe de maintien 38 d'une pompe 34. Cette pompe plonge dans la chambre 1 en traversant la chambre 2 au travers d'une cheminée intérieure 35 se raccordant de manière étanche à ladite pompe. Cette pompe 34 est du type connu dans les diffuseurs, dispenseurs et analogues, comportant un tube plongeur 36, une pièce d'appui 37 enfonçable, et un système mécanique intérieur grâce auquel un enfoncement de la pièce d'appui 37 provoque un effet de pompage, avec expulsion du produit pompé au travers de la pièce d'appui 37. De telles pompes sont commercialisées, par exemple, sous les marques "Mistette Mark II" et "Mistette 08" par la Société Calmar-Albert, ou, sous la référence SFV par la Société Française d'Aérosol et de Bouchage. Un bec 39 coiffe la pièce d'appui 37, de manière que le liquide dispensé pénètre dans le tube d'injection 4, et à pouvoir être enfoncé par rapport au corps de pompe 34.

Le liquide usé retournant dans le tuyau 5 débouche sous le bec et s'écoule autour du corps de la pompe 34, pour pénétrer finalement dans le flacon 32 grâce à des orifices 40 prévus dans la partie de rattachement de la jupe 38 au corps de pompe 34.

Le dispositif des figures 7 à 9 est plus spécialement conçu en tant que dispositif jetable, non rechargeable. La chambre 1 de liquide à injecter y est formée par un récipient cylindrique 50 à lèvre d'étanchéïté 58, dans lequel peut coulisser de manière étanche la tête 51 d'un piston 52 comportant une tige creuse 53 débouchant à la base du piston et s'emmanchant dans un diffuseur 54. Ce diffuseur 54 comporte d'une part une paroi extérieure cylindrique 55 bordée d'une collerette 59 venant coiffer le récipient inférieur 50, et intégre la sonde rigide 3 semblable à celle des dispositifs des figures 4 à 6. Le tube d'injection est relié à la tige creuse 53, tandis que le tuyau de récupération laisse retomber le liquide usée dans le volume intérieur du diffuseur 54. Il suffit d'appuyer sur le diffuseur 54 par rapport au récipient 50 (comme montrer sur la figure 10) pour chasser le liquide de la chambre 1 et le faire retourner après usage dans la chambre 2. La hauteur du récipient 50 est égale ou inférieure à la profondeur du diffuseur 54 dans lequel il s'enfonce de manière à rendre difficile son extraction du diffuseur après utilisation complète et éviter les tentatives de réutilisation non contrôlée.

Le mode de réalisation des figures 10 et 11 se distingue du précédent en ce que le diffuseur 60 s'enfonce dans le récipient 61 au lieu de l'inverse. Le récipient 61 est bordé par une collerette 62.

Comme indiqué plus haut, le liquide actif utilisé conformément à l'invention peut être de l'eau, du sérum physiologique, ou, plus avantageusement encore, une solution d'un antiseptique local, tel que le "Mercryl Laurylé" ou un antiseptique de type ammonium quaternaire, phénol tel que le thymol ou salol, chlorhéxidine ou autre.

**Revendications**

1. Dispositif pour le nettoyage d'une cavité auriculaire par injection d'un liquide propre et récupération du liquide usé, comprenant :

un injecteur présentant une pompe et un conduit d'injection (4) ayant un orifice de sortie et destiné à amener le liquide propre à la cavité,

une première chambre (1) destinée à contenir le liquide propre et reliée au conduit d'injection (4),

un conduit (5) de récupération du liquide usé ayant un orifice d'entrée (12) et,

une seconde chambre (2) destinée à recevoir le liquide usé récupéré par le conduit (5) de récupération,

caractérisé en ce que le conduit (5) de récupération est agencé de sorte que le liquide usé s'écoule par gravité de ladite cavité à travers ce dernier jusqu'à ladite seconde chambre (2) et présente une section supérieure à celle du conduit d'injection (4), et en ce qu'il comprend en outre,

un moyen d'étanchéité (9,23) de ladite cavité, moyen traversé par le conduit d'injection (4) et par le conduit (5) de récupération, l'orifice de sortie du conduit d'injection (4) et l'orifice d'entrée (12) du conduit (5) étant sensiblement adjacents et débouchant à l'intérieur de la cavité, lorsque le dispositif est en position de nettoyage.

2. Dispositif selon la revendication 1, caractérisé en ce que le conduit (5) de récupération et le conduit (4) d'injection sont placés intérieurement l'un à l'autre, au moins au niveau de leurs orifices respectifs d'entrée et de sortie.

3. Dispositif selon la revendication 1, caractérisé en ce que le conduit de récupération (5) et le conduit d'injection (4) sont placés de manière contiguë, au moins au niveau de leurs orifices respectifs d'entrée et de sortie.

4. Dispositif selon la revendication 1, caractérisé en ce que le conduit de récupération (5) et le conduit d'injection (4) sont adjacents au moins sur une partie terminale en amont du moyen d'étanchéité.

5. Dispositif selon l'une quelconque des revendications 1 à 4, caractérisé en ce que le moyen d'étanchéité (9) est constitué par une jupe en paroi souple susceptible de se plaquer contre la cavité auriculaire sous l'effet de la pression du liquide injecté.

6. Dispositif selon l'une quelconque des revendications 1 à 4, caractérisé en ce que le moyen d'étanchéité (23) est constitué par un bouchon en matériau légèrement élastique.

7. Dispositif selon l'une quelconque des revendications 1 à 6, caractérisé en ce que la première chambre (1) constitue la pompe et comprend une enceinte à volume déformable.

8. Dispositif selon la revendication 7, caractérisé en ce que l'enceinte est à parois déformables (6).

9. Dispositif selon la revendication 7, caractérisé en ce que l'enceinte est fermée par un piston (52) déplaçable dans l'enceinte.

**Patentansprüche**

1. Vorrichtung zur Reinigung eines aurikulären Hohlraums durch Einspritzen einer geeigneten Flüssigkeit und Rückführung der verwendeten Flüssigkeit, mit:

einer Einspritzdüse, die eine Pumpe und eine Einspritzleitung (4), die eine Ausgangsöffnung besitzt und dazu vorgesehen ist, dem Hohlraum die geeignete Flüssigkeit zuzuführen, aufweist,

einer ersten Kammer (I), die dazu vorgesehen ist, die geeignete Flüssigkeit aufzunehmen, und die mit der Einspritzleitung (4) verbunden ist,

einer Leitung (5) zur Rückführung der verwendeten Flüssigkeit, die eine Eingangsöffnung (12) besitzt, und

einer zweiten Kammer (2), die dazu vorgesehen ist, die rückgeführte verwendete Flüssigkeit von der Rückführungsleitung (5) zu aufzunehmen,

dadurch gekennzeichnet, daß die Rückführungsleitung (5) so angeordnet ist, daß die verwendete Flüssigkeit durch die Schwerkraft vom Hohlraum durch diesen letzteren bis zur zweiten Kammer (2) fließt, und einen Abschnitt oberhalb der Einspritzleitung (4) aufweist und daß sie außerdem

ein Mittel (9, 23) zum Abdichten des Hohlraums, das von der Einspritzleitung (4) und von der Rückführungsleitung (5) durchsetzt wird, umfaßt, wobei die Ausgangsöffnung der Einspritzleitung (4) und die Eingangsöffnung (12) der Leitung (5) im wesentlichen benachbart sind und in das Innere des Hohlraums münden, wenn sich die Vorrichtung in der Reinigungsposition befindet.

2. Vorrichtung gemäß Anspruch 1, dadurch gekennzeichnet, daß die Leitung (5) zur Rückführung und die Leitung (4) zum Einspritzen wenigstens auf Höhe ihrer Eingangs- bzw. Ausgangsöffnungen ineinander angeordnet sind.

3. Vorrichtung gemäß Anspruch 1, dadurch gekennzeichnet, daß die Rückführungsleitung (5) und die Einspritzleitung (4) wenigstens auf Höhe ihrer Eingangsbzw. Ausgangsöffnung auf aneinandergrenzende Weise angeordnet sind.

4. Vorrichtung gemäß Anspruch 1, dadurch gekennzeichnet, daß die Rückführungsleitung (5) und die Einspritzleitung (4) wenigstens in einem Endbereich vor dem Dichtungsmittel benachbart sind.

5. Vorrichtung gemäß einem der Ansprüche 1 bis 4, dadurch gekennzeichnet, daß das Dichtungsmittel (9) von einem Mantel mit biegsamer Wand gebildet wird, der dazu geeignet ist, sich unter der Wirkung des Drucks der eingespritzten Flüssigkeit an den aurikulären Hohlraum schmiegen.

6. Vorrichtung gemäß einem der Ansprüche 1 bis 4, dadurch gekennzeichnet, daß das Dichtungsmittel (23) von einem Stopfen aus einem etwas elastischen Material gebildet wird.

7. Vorrichtung gemäß einem der Ansprüche 1 bis 6, dadurch gekennzeichnet, daß die erste Kammer (I) die Pumpe bildet und einen Behälter mit verformbarem Volumen umfaßt.

8. Vorrichtung gemäß Anspruch 7, dadurch gekennzeichnet, daß der Behälter verformbare Wände (6) besitzt.

9. Vorrichtung gemäß Anspruch 7, dadurch gekennzeichnet, daß der Behälter von einem Kolben (52) verschlossen wird, der im Behälter verschiebbar ist.

## Claims

1. Device for cleaning the auricular cavity by injecting a clean liquid and recovering the waste liquid, comprising:
an injector provided with a pump and an injection tube (4) with an outlet orifice adapted to bring the clean liquid into the cavity,
a first chamber (1) designed to contain the clean liquid and connected with the injection tube (4),
a tube (5) for recovery of the waste liquid, provided with an inlet orifice (12), and
a second chamber (2) designed to receive the waste liquid recovered by the recovery tube (5),
characterized in that the recovery tube (5) is so arranged that the waste liquid flows by gravity from said cavity through the latter to reach said second chamber (2) and has a cross-section greater than that of the injection tube (4), and in that it further comprises,
sealing means (9, 23) for sealing said cavity, through which means pass the injection tube (4) and the recovery tube (5), the outlet orifice of the injection tube (4) and the inlet orifice (12) of the tube (5) being substantially adjacent and issuing into the cavity, when the device is in cleaning position.

2. Device according to claim 1, characterized in that the recovery tube (5) and the injection tube (4) are placed one inside the other, at least at the level of their respective inlet and outlet orifices.

3. Device according to claim 1, characterized in that the recovery tube (5) and the injection tube (4) are placed in contiguous manner at least at the level of their respective inlet and outlet orifices.

4. Device according to claim 1, characterized in that the recovery tube (5) and the injection tube (4) are adjacent at least over an end portion upstream of the sealing means.

5. Device according to any one of claims 1 to 4, characterized in that the sealing means (9) is constituted by a skirt having a flexible wall, adapted to be applied against the auricular cavity under the effect of the pressure of the injected liquid.

6. Device according to any one of claims 1 to 4, characterized in that the sealing means (23) is constituted by a plug in slightly resilient material.

7. Device according to any one of claims 1 to 6, characterized in that the first chamber (1) constitutes the pump and comprises an enclosure whose volume is deformable.

8. Device according to claim 7, characterized in that the enclosure has deformable walls (6).

9. Device according to claim 7, characterized in that the enclosure is closed off by a piston (52) movable in the enclosure.

Fig. 1

Fig. 2

Fig. 3

# Fig. 4

1

20

4

24

22

26

5

21

23

25

2

# Fig. 5

21
20

4

28

5

22

27

Fig.6

39

37

4

5

34

40

38

33

35

_2_

32

31

36

_1_

30

Fig.7

Fig.8

Fig.9

Fig.10

Fig.11